# EUROPEAN PATENT SPECIFICATION

(11) **EP 0 535 347 B1**
(45) Date of publication and mention of the grant of the patent: **02.10.1996**
(21) Application number: 92114079.4
(22) Date of filing: 18.08.1992
(51) Int. Cl.: C12P 21/08, C12N 5/20, G01N 33/577, G01N 33/543

(54) **Monoclonal antibodies to sulfonated polymers**
Monoklonale Antikörper gegen sulfonierte Polymere
Anticorps monoclonaux contre des polymères sulphonés

(30) Priority: 30.08.1991 US 752746
(43) Date of publication of application: 07.04.1993
(73) Proprietor: NALCO CHEMICAL COMPANY, Naperville Illinois 60563-1198 (US)
(72) Inventor: Wetegrove, Robert L., Winfield, IL 60190 (US); Wu, Ming-Hsien, Los Angeles, CA 90049 (US); Balakrishnan, Krishna, Richmond, CA 94805 (US)
(74) Representative: Ruschke, Olaf

(56) References cited:
- EP-A- 0 244 584
- WO-A-90/06954
- US-A- 4 756 881

## Description

### Field of the Invention

The invention relates generally to antibody assays, and more particularly, to monoclonal antibodies and antibody assays for the detection of sulfonated copolymers of acrylic acid and acrylamide, as well as to methods of manufacturing the antibodies.

### Description of the Prior Art

As described in U.S.-A-4,756,881 and 4,752,443, water-soluble sulfonated copolymers of acrylic acid and acrylamide (hereinafter referred to as "sulfonated copolymers") are used in the treatment of industrial cooling water to prevent corrosion and mineral deposits (scale). Generally, the active sulfonated copolymers remove dissolved minerals from the cooling water by complexing with the mineral. Over time, the complexation sites of the sulfonated copolymer molecules become saturated and the copolymer molecules become inactive, unable to remove any additional minerals from the cooling water.

To prevent corrosion and scale damage to machinery, as the polymers are inactivated they must be removed and replaced by active sulfonated copolymer. Thus, active sulfonated copolymer must be continually fed into the cooling water to replace the inactive sulfonated copolymer. Maintaining the proper feed level for the active sulfonated copolymer is essential for optimum performance of the cooling water system. An improper feed rate can lead to serious problems. For example, insufficient active sulfonated copolymer can result in the water treatment being overwhelmed by dissolved minerals, thereby causing severe corrosion or scale deposit. On the other hand, maintaining too high a level of the active polymer is very expensive and is an inefficient method for treating industrial cooling water.

Although several methods are available for determining the total concentration of sulfonated copolymer in an industrial cooling water system, i.e., active plus inactive sulfonated copolymer, these techniques are unsatisfactory since they only determine the concentration of total sulfonated copolymer, and do not measure the concentration of the active sulfonated copolymer. Moreover, these methods suffer from lack of specificity or poor sensitivity. For example, the older methods for detecting sulfonated copolymers include colloid titration with PVSK, complexation with hyamine 1622, or reaction of excess magnesium with chrome azurol S. The above tests detect any polyanionic material and have a detection threshold of about 50 ppm polymer. Presently, the total amount of active sulfonated polymer in an industrial cooling water system cannot be inexpensively and rapidly determined.

### Brief Description of the Drawings

Fig. 1 is a graphic illustration of the binding profile of the monoclonal antibody from a hybridoma cell line (6E2-H1-G4) to the sulfonated copolymer of acrylic acid and acrylamide conjugated to bovine serum albumin (BSA-SCP) +, the non-sulfonated copolymer of acrylic acid and acrylamide conjugated to bovine serum albumin (BSA-CP) -, the sulfonated copolymer of acrylic acid and acrylamide (SCP) -■-, and to a control bovine serum albumin (BSA) -□-;
Fig. 2 graphically illustrates the binding profile of the monoclonal antibody from another hybridoma cell line (6D12-H9-H3) to BSA-SCP +, BSA-CP-, SCP -■-, and BSA -□-;
Fig. 3 is a graphic illustration of the binding profile of the monoclonal antibody from a further hybridoma cell line (4D4-C9-F6) to BSA-SCP+, BSA-CP-, SCP -■-, and BSA -□-;
Fig. 4 is a graphic illustration of an inhibition Enzyme Linked Immunosorbent Assay (ELISA) assay using the monoclonal antibody from the hybridoma cell line 6D12-H9-H3, wherein -■- is BSA, + is CP, ≠ is SCP, and -□- is BSA-SCP;
Fig.5 is a graphic illustration of an inhibition ELISA assay using the monoclonal antibody from the hybridoma cell line 6E2-H1-G4, wherein -■- is BSA, - is CP, + is SCP, and -□- is BSA-SCP;
Fig. 6 is a graphic illustration of an inhibition ELISA assay using the monoclonal antibody from the hybridoma cell line 6D12-H9-H3 to detect active and inactive SCP in Phosphate Buffered Saline in (PBS) buffer, wherein group 1 includes only PBS, group 2 includes 10 ppm CP, group 3 includes 10 ppm SCP, group 4 includes 20 ppm SCP, group 5 includes 10 ppm SCP, and group 6 includes 10 ppm inactive SCP and 4mMFe; and
Fig. 7 is a graphic illustration of an inhibition ELISA assay using the monoclonal antibody from the hybridoma cell line 6D12-H9-H3 to detect active and inactive SCP in Tris Buffered Saline (TBS) buffer, wherein group 1 includes only TBS, group 2 includes 10 ppm CP, group 3 includes 10 ppm SCP, group 4 includes 20 ppm SCP, group 5 includes 10 ppm SCP, and group 6 includes 10 ppm inactive SCP and 4mMFe.

### Summary of the Invention

One aspect of the present invention is directed to a monoclonal antibody having an affinity to sulfonated copolymers of acrylic acid and acrylamide. The monoclonal antibodies of the present invention have an absolute specificity for the sulfonated acrylic acid-acrylamide copolymer and a lower limit of detection in the femtogram per ml range when used in some detection formats.

The monoclonal antibodies of the invention are produced by hybridoma cell lines. One preferred hybridoma cell line is 6E2-H1-G4. Another preferred hybridoma cell line is 6D12-H9-H3.

Another aspect of the invention is directed to a method of manufacturing the above monoclonal antibody having an affinity to a sulfonated copolymer of acrylic acid and acrylamide. The inventive method including the steps of: a) immunizing a mammal, preferably a moise, with the sulfonated copolymer of acrylic acid and acrylamide attached to a carrier protein; b) preparing a hybridoma cell producing the monoclonal antibody from cells removed from the immunized mammal; c) cloning the hybridoma cell to produce a hybridoma cell line; and d) extracting the monoclonal antibody from the hybridoma cell line. Accordingly, the mammal is preferably a mouse and the hybridoma cell line is 6E2-H1-G4 or 6D12-H9-H3.

A further aspect of the invention is directed to a process for the determination of the presence or concentration of a sulfonated copolymer of acrylic acid and acrylamide in a fluid. The inventive process including the step of incubating a sample of the fluid containing the sulfonated copolymer of acrylic acid and acrylamide with a monoclonal antibody as described above having an affinity for the sulfonated copolymer of acrylic acid and acrylamide, the monoclonal antibody being bound to a solid carrier.

### Description of the Preferred Embodiments

Hybridoma cell lines producing monoclonal antibodies having an affinity for sulfonated copolymers of acrylic acid and acrylamide (SCP) have been invented and are herein described and claimed. The monoclonal antibodies of the invention bind to SCP with varying degrees of specificity. By using two different assay formats, it has been conclusively proven that these monoclonal antibodies can recognize SCP at concentrations ranging from zero to a few hundred parts per million. Examples described herein demonstrate the feasibility of using the monoclonal antibodies of the invention to determine the presence or concentration of SCP in fluids, such as water samples from industrial cooling water towers.

Accordingly to one embodiment of the invention, a monoclonal antibody having an affinity to sulfonated copolymer is used in an Enzyme Linked Immunosorbent Assay (ELISA) for determining the concentration of SCP in a fluid sample. ELISA formats using the monoclonal antibodies of the invention are preferred for measuring the concentration of SCP in fluid samples. Three preferred immunoassay strategies are sandwich ELISA, competition ELISA and indirect ELISA. Although several antibodies described herein are useful in assaying SCP in a fluid sample, the two most preferred monoclonal antibodies are those produced by hybridoma cell lines 6E2-H1-G4 and 6D12-H9-H3 since these two clones have shown the greatest anti-SCP specificity in indirect ELISAs.

According to one preferred embodiment of the invention a sandwich ELISA will be used for measuring the concentration of SCP. Preferably, purified anti-SCP antibody will be used as a capture antibody. The antibody will be adsorbed to wells of 96-well microtiter plates. Test samples containing SCP will be added and allowed to bind to the capture antibody. In the next step, enzyme-labelled anti-SCP antibody will be added and allowed to bind to the exposed antigenic sites on the SCP molecules captured by the coating antibody. The amount of enzyme labelled antibody retained after washing would therefore correlate directly with the amount of SCP in the sample. The level of enzyme label bound in each well will be quantitated by incubation with a chromogenic enzyme substrate and measurement of the resulting color change. A standard curve will be generated using known concentrations of SCP polymer. This approach has the advantage of higher sensitivity normally associated with sandwich ELISAs.

In another preferred embodiment, a competition ELISA is used to measure the SCP for a fluid sample. Accordingly to this embodiment, purified anti-SCP antibody will be adsorbed onto wells of 96-well microtiter plates. Test samples containing SCP will be added to the plates with known quantities of enzyme-labelled SCP polymer. The amount of enzyme-labelled SCP retained after washing will be inversely proportional to the amount of SCP originally present in the test samples. The amount of enzyme present will be measured by the addition of a suitable chromogenic substrate. Standard curves will be generated using known quantities of SCP. This particular format has the advantage of being essentially a one-step assay with only one incubation period.

According to a still further embodiment of the invention, indirect ELISA assay is utilized to measure the concentration of SCP in a fluid. According to the embodiment, known quantities of Bovine Serum Albumin (BSA-SCP) conjugate will be adsorbed to replicate wells of 96 well microtiter plates. Next, various dilutions of the anti-SCP monoclonal antibody will be allowed to bind to the immobilized SCP. Enzyme-labelled goat anti-mouse Ig will be added next and allowed to bind to the primary antibody. A chromogenic substrate will then be added which will be converted to a colored product by the bound enzyme. The resultant color change will be quantified by measuring the absorbance (optical density) at 492 nm. The amount of color change will be proportional to the amount of enzyme-labelled antibody retained and thus will correlate directly with the amount of anti-SCP antibody which was able to initially bind to the immobilized SCP. The results of this experiment will yield the optimal concentration of anti-SCP antibody to use in the assay.

Having defined the appropriate concentration of antibody for an indirect ELISA, inhibition of binding between the monoclonal antibody and the SCP-coated microtiter plates by SCP present in solution will be determined. The monoclonal anti-SCP primary antibody will be incubated with various concentrations of SCP before being added to the SCP-coated microtiter plates. A standard curve will then be prepared by plotting the percentage inhibition as a function of free SCP concentration. This will be carried out for each fixed quantity of immobilized SCP and the primary antibody. This assay format will be suitable if this standard curve shows a steep dose-response at the concentrations that are relevant to those which occur in cooling water samples. It has the advantage of not requiring any additional purification or modification of the anti-SCP monoclonal antibody.

Any of the assays discussed above could be further developed and refined using other solid support systems such as coated tubes and polymer membranes. However, the sandwich ELISA or the competition ELISA formats are preferred since they could be designed as a "dipstick" assay. Nevertheless, any of the above assay formats are intended to be used with the monoclonal antibodies of the present invention to measure the concentration of SCP in a fluid sample.

The following examples are presented to describe preferred embodiments and utilities of the invention.

### Example 1: Antigen Synthesis

The antigens were prepared as follows:

Twenty mg of the polymer was first derivatized with the coupling agent, 1-ethyl 3(3'-dimethylamino-propyl) carbodiimide (EDC) at a pH of 5.0. After a 15 minute incubation at room temperature, 15 mg of Keyhole Limpet Hemocyanin (KLH) or 10 mg of Bovine Serum Albumin (BSA) was added and allowed to react at pH 7.0 for 4 hours, also at room temperature. Small molecular weight side products were removed by overnight dialysis at 4°C.

Both sulfonated (SCP) and non-sulfonated (CP) forms of the acrylate-acrylamide copolymers were tested. The sulfonated copolymer was obtained from Nalco Chemical Company under the tradename designation PRISM®. The non-sulfonated copolymer was also obtained from Nalco Chemical Company. High and low molecular weight fragments were tested for both forms. Exact molecular weights of the copolymers were 6.6 kD and 23 kD for the non-sulfonated forms and 7.4 kD and 26 kD for the sulfonated forms. The smaller molecular weight copolymers, both sulfonated and non-sulfonated, were conjugated through carboxylic acid residues on the copolymers to amino groups on the carrier proteins KLH and BSA using the coupling agent EDC as described above. Gel electrophoresis of the BSA conjugate indicated successful conjugation with approximately one to four copolymer molecules conjugated per molecule of BSA. The KLH conjugates were too heterogeneous and less well defined to be meaningfully analyzed by electrophoresis.

### Example 2: Utility SCP-BSA conjugate in Indirect ELISA Screening.

The BSA conjugates of the lower molecular weight copolymers were tested for adsorption onto microtiter plates by performing an indirect ELISA using rabbit anti-BSA antiserum. Plates were coated with the conjugates at a concentration of 0.01 mg/ml (0.5 µg/well) in phosphate-buffered saline, pH 7.2 (PBS). Unoccupied sites on the plates were blocked with a 5% solution of non-fat milk in PBS. Dilutions of rabbit anti-BSA antiserum were incubated with the plate. Horseradish peroxidase (HRP) labelled goat anti-rabbit antibody was then allowed to bind to the primary anti-BSA antibody. The bound enzyme (HRP) was then quantitated with a chromogenic substrate. These assays were performed in duplicate, the results are tabulated in Table 1. These results clearly indicate that the BSA conjugates bound strongly to the ELISA plates, thus confirming the utility of these conjugates for the screening of anti-SCP antibodies in an indirect ELISA format.

**TABLE 1**

| Indirect ELISA with Rb anti-BSA sera | | | |
|---|---|---|---|
| Set | Antigen | OD_{1/2max} | Titer value |
| #1 | SCP-BSA | 0.96 | 1:9000 |
| | CP-BSA | 0.94 | 1:7100 |
| | BSA | 0.87 | 1:14,000 |
| | No Ag | 0.31 | 1:50 |
| #2 | SCP-BSA | 0.94 | 1:9000 |
| | CP-BSA | 0.92 | 1:7700 |
| | BSA | 0.86 | 1:13,000 |
| | No Ag | 0.36 | <1:50 |

### Example 3: Immunization

Immunization of four groups (A-D) of female Balb/c mice, five mice per group, approximately 9 - 12 weeks of age, was performed. Groups A and B were boosted weekly with 100 µg of SCP-KLH immunogen per mouse administered via intraperitoneal injection. Group A received immunogen emulsified in Freund's adjuvant, Group B received immunogen emulsified in Ribi's adjuvant. Groups C and D received weekly immunizations of 100 ug of unconjugated SCP immunogen per mouse emulsified in Freund's adjuvant or Ribi's adjuvant respectively. Mice were bled periodically and each serum sample was evaluated by indirect ELISA against SCP-BSA and CP-BSA. Antibody titer values were determined from the ELISA results; antibody titer being defined as the dilution of the sample sera which yields an optical density that is one-half of the maximum signal obtained (OD_{1/2max}) in an indirect ELISA. Table 1 presents the titer results obtained for all four groups of animals.

### Example 4: Fusion

Mice with elevated antibody titer against SCP-BSA were sacrificed and the splenocytes were isolated and fused with Hypoxanthine Guanidine Phosphoribosyl Transferase (HGPRT) deficient SP2/0 plasmacytoma cells utilizing polyethylene glycol (PEG) as the fusing agent. Fusion #1 was performed with the two mice from Group B which had titers of 1:11,000 and 1:14,000, respectively, against SCP-BSA. Their corresponding titer values against CP-BSA were 1:4700 and 1:6700. Fusion #2 was performed using two mice from Group E (an additional group of mice which was subsequently started) with anti-SCP-BSA titer values of 1:2600 and 1:2900 respectively. Their corresponding titer values against CP-BSA were 1:1000 and 1:920. The fused cells were plated into 96-well tissue culture plates and the resultant hybridomas were identified by selection in Hypoxanthine Aminopterin Thymidine (HAT) medium. The hybridoma cell lines developed which produced anti-SCP monoclonal antibodies were 6E2-H1-G4, 6D12-H9-H3, 6C8-F1-F9, 4D4-C9-F6, 4B1-H6-E10 and 4F12-C12.

### Example 5. Antibody Scale-up

Hybridoma cell lines 6E2-H1-G4 and 6D12-H9-H3 were scaled up by growing them in vitro for several passages and injecting them into a group of pristane-primed Balb/c mice. The cells grew as ascites tumors in these mice and the resultant fluid accumulated in their peritoneal cavities was harvested. The ascites fluid was enriched in the desired anti-SCP antibody which was purified and used in the assays described below.

### Example 6. Antibody Purification

Depending on the assay format being pursued, antibodies will be required either as unpurified ascites or as a highly purified immunoglobulin preparation. Antibodies were purified from ascites fluid by a variety of methods, depending on the antibody isotype and the sensitivity of the antibody to various buffers. The clones 6E2-H1-G4 and 6D12-H9-H3 identified in Example 5 are of the IgG isotype, and accordingly, they were purified by using the pseudo-affinity matrix Protein A.

### Example 7. Monoclonal Antibodies Specific For SCP

Table 2 summarizes the binding data for the different hybridoma cell lines that were developed. All the cell lines were subcloned by limiting dilution in the presence of appropriate growth factors. When clonal populations of cells were identified, the supernatants were assayed again for SCP binding and single colonies that exhibited the required binding specificity were expanded and frozen down.

**TABLE 2**

| ANTI-SCP HYBRIDOMAS, BINDING DATA | | | | | |
|---|---|---|---|---|---|
| No. | Cell line | ELISA Signal of parent | | ELISA Signal of clone | |
| | | SCP | CP | SCP | CP |
| 1 | 6D12 H9-H3 | 1.70 | 0.11 | 1.48 | 0.02 |
| 2 | 6C8-F1-F9 | 0.92 | 0.02 | 0.77 | 0.00 |
| 3 | 4D4-C9-F6 | 0.77 | 0.18 | 0.61 | 0.12 |
| 4 | 6E2-H1-G4 | 2.00 | 0.11 | 1.81 | 0.45 |
| 5 | 4B1-H6-E10 | 1.39 | 0.50 | 1.74 | 0.55 |
| 6 | 4F12-C12 | 1.46 | 0.16 | 1.48 | 1.38 |

### Example 8. Cross-reactivity Profile of the Anti-SCP Monoclonal Antibodies

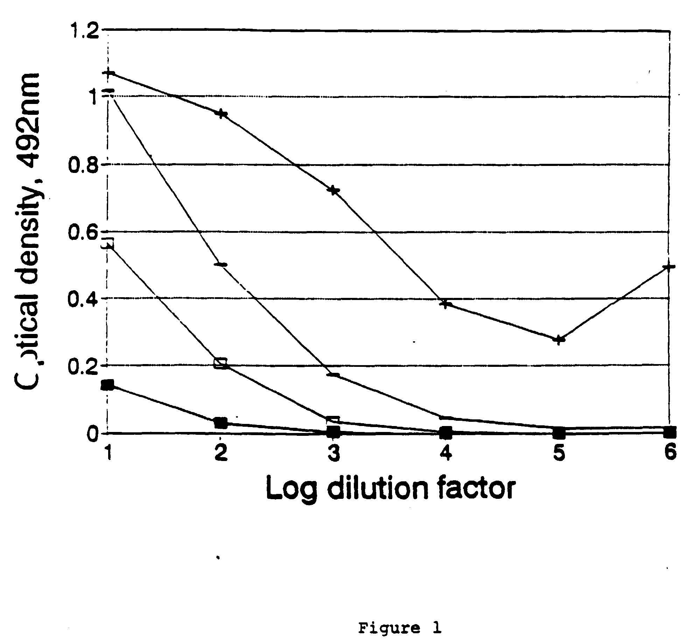

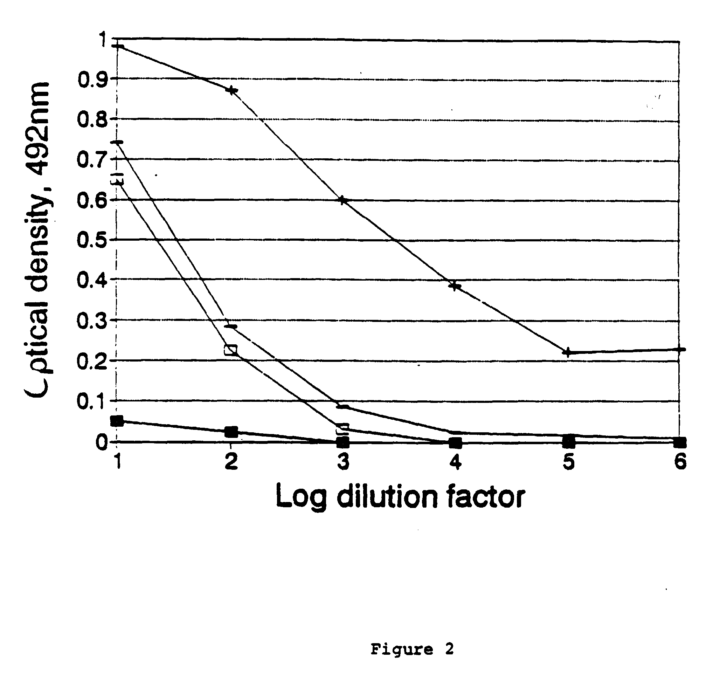

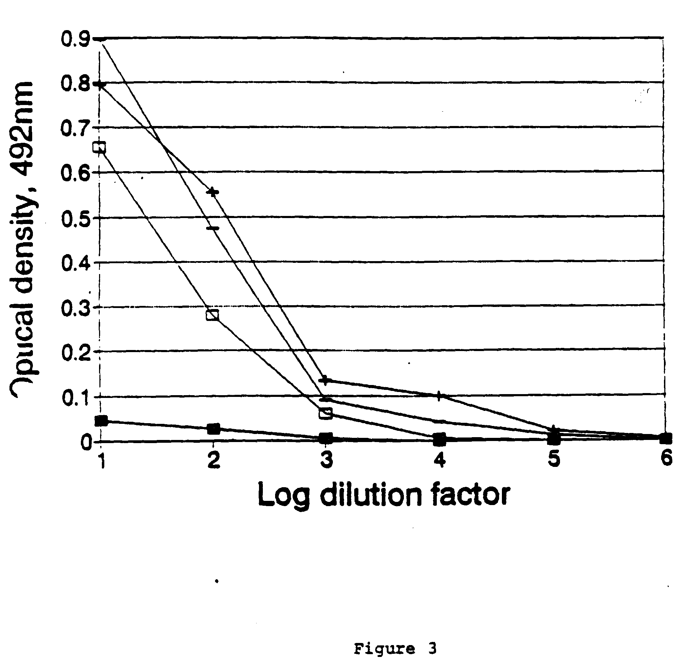

The binding specificity of the different anti-SCP monoclonal antibodies was tested by an indirect ELISA. Replicate wells were coated with 500 ng per well of the following panel of antigens: BSA-SCP, BSA-CP, SCP or BSA. Serial dilutions of the ascites fluid ranging from 1:10 to 1:10⁶ in steps of 10 were incubated with the various antigen coated plates. After an incubation with the ascites fluid samples, the plates were washed and incubated with HRP-labelled goat anti-mouse Ig. Finally, the quantity of enzyme (HRP) retained in each well was quantified using H₂O₂ and a chromogenic substrate. The optical density readout at 492 nm was recorded using an automated ELISA reader and the results are summarized in Figs. 1, 2, and 3.

Of the three different monoclonal antibodies whose results are shown in Figs. 1, 2, and 3, cell lines 6E2-H1-G4 and 6D12-H9-H3 show a dramatically higher binding specificity for BSA-SCP as compared to BSA-CP whereas cell line 4D4-C9-F6 is almost equally reactive to BSA-SCP and BSA-CP. These results confirm the earlier findings shown in Table 1, where these studies were carried out with spent tissue culture supernatants from these cell lines.

### Example 9. Specificity of Anti-SCP Monoclonal Antibodies 6E2-H1-G4 and 6D12-H9-H3 as Evaluated By Inhibition Assays:

Based on the information obtained from the indirect ELISA results, summarized in Figs. 1, 2, and 3, two of the cell lines 6E2-H1-G4 and 6D12-H9-H3 which produced monoclonal antibody that showed specificity to BSA-SCP are the preferred cell lines. The results for the indirect ELISA were used to calculate the optimal concentrations of the two different monoclonal antibodies which would produce the most sensitive results in an inhibition ELISA. The inhibition ELISA carried out in this set of experiments is briefly described below.

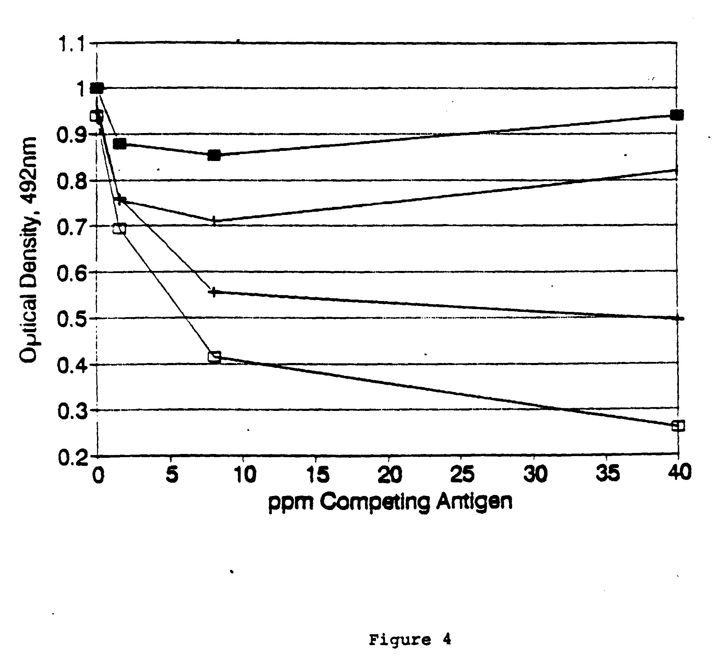

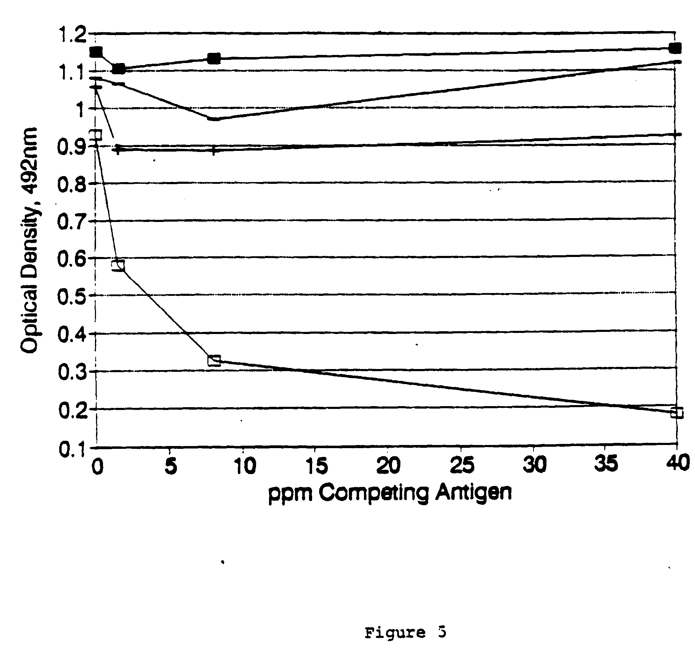

Microtiter plates were coated with 500 ng per well of BSA-SCP and nonspecific sites on the plate were blocked. Replicate samples of the test ascites fluid at a fixed concentration were preincubated with increasing concentrations of a panel of competing antigens for 2 h at room temperature. After this preincubation, the antibodies were allowed to bind to the BSA-SCP coated plates. The panel of antigens used in the competition ELISA included BSA-SCP, SCP, CP or BSA. The object of the assay was to demonstrate the antibody specificity in a solution phase assay. A highly specific antibody would bind tightly with the cognate antigen in solution and not the others. This would result in an inhibition of the antibody from binding to the BSA-SCP coated plate only in the presence of the appropriate antigen and not the others. The quantity of anti-SCP monoclonal antibody bound to the BSA-SCP coated plate was determined as before with the use of an HRP-labelled goat anti-mouse Ig and a chromogenic substrate for HRP. Results of such assays are shown in Figs. 4 and 5. As seen from these figures, the two cell lines 6E2-H9-G4 and 6D12-H9-H3 do show considerable differences in the inhibition ELISA. Cell line 6D12-H9-H3 (shown in Fig. 4) detects approximately 5 to 40 ppm of SCP and distinguish it from CP. Cell line 6E2-H1-G4, as shown in Fig. 5, was not as sensitive, but could still be useful in an assay. All of the above assays were performed in phosphate buffered saline, pH 7.2.

### Example 10. Assay of Simulated Field Samples:

Various aqueous samples containing SCP, acquired from Nalco Chemical Company, Naperville, Illinois, under the trademark designation PRISM® polymer, were prepared. Some of the samples mimicked "field" conditions in that they contained fairly high concentration of Fe⁺³ and Ca⁺² ions such that the SCP was substantially inactive. Also tested were samples which contained quantities of uncomplexed active SCP sufficient enough to prevent scaling.

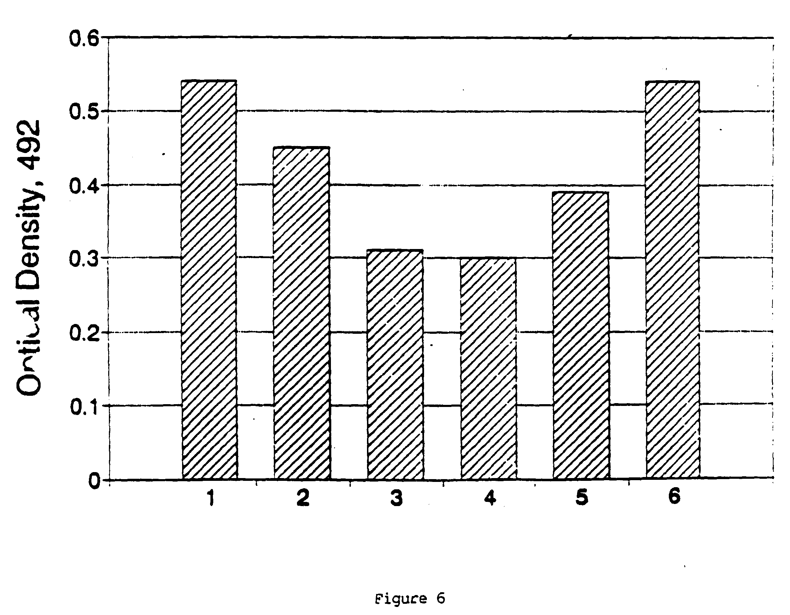

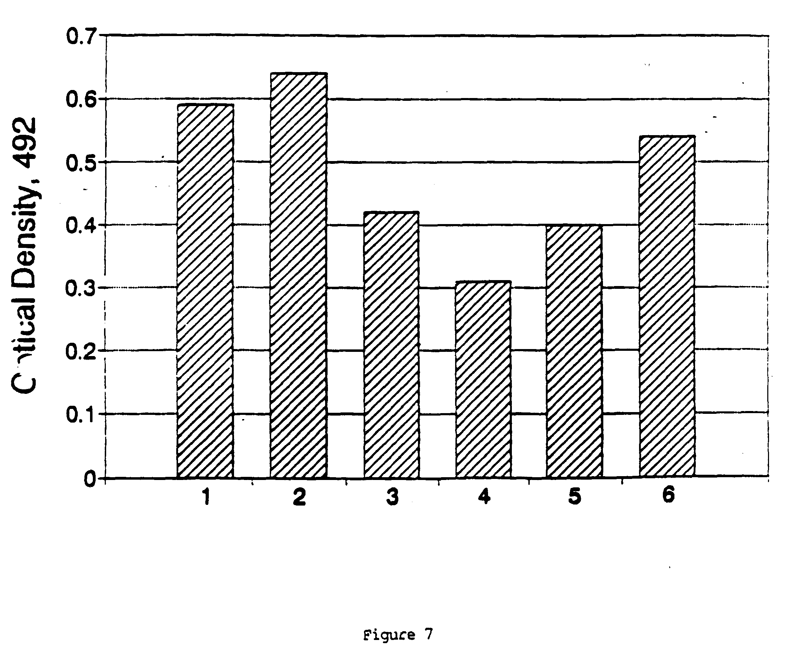

A series of experiments were carried out to determine if the monoclonal antibodies of the invention could distinguish between an "active" and an "inactive" solution of SCP. Results of these experiments are shown in Figs. 6 and 7. Parallel experiments were also carried out using phosphate buffered saline (PBS) and Tris buffered saline (TBS). As seen from Figs. 6 and 7, the assay effectively distinguished between an "active" sample (sample 4) and an "inactive" sample (sample 6). In experiments shown in Fig. 6, the assays were carried out in PBS, whereas those shown in Fig. 7 utilized TBS. Samples 3 and 2, the positive and negative controls, contained 10 ppm of the SCP polymer and CP polymer respectively, and were made up in the respective buffers PBS and TBS. The samples were all diluted 1:2 with the appropriate buffer in the assay, either PBS or TBS and were composed of the following: Sample 1 included only PBS. Sample 4 contained 20 ppm of SCP and hence should be an "active" sample, in as much as it contained quantities of SCP sufficient enough to prevent scaling. Sample 5 contained 10 ppm of SCP and could also be considered "active" although with a lower polymer concentration. Sample 6 was prepared with 10 ppm SCP and 4 mM Fe⁺³ in such a way to simulate an "inactive" sample. Sample 6 presumably will not prevent scaling and clearly could be identified as not containing any detectable quantity of free, active SCP.

## Claims

1. A monoclonal antibody having an affinity for a sulfonated copolymer of acrylic acid and acrylamide.

2. The monoclonal antibody of claim 1, wherein said monoclonal antibody is produced by a hybridoma cell line.

3. A method of manufacturing a monoclonal antibody according to claim 1 having an affinity for a sulfonated copolymer of acrylic acid and acrylamide, the method including the steps of:
a) immunizing a mammal with the sulfonated copolymer of acrylic acid and acrylamide;
b) preparing a hybridoma cell producing the monoclonal antibody from a cell from the immunized mammal;
c) cloning said hybridoma cell to produce a cell line; and
d) extracting said monoclonal antibody from said hybridoma cell line.

4. The method of claim 3 wherein the mammal is a mouse.

5. A process for the determination of the presence or concentration of a sulfonated copolymer of acrylic acid and acrylamide in a fluid, the process including the step of
incubating a sample of the fluid containing the sulfonated copolymer of acrylic acid and acrylamide with a monoclonal antibody according to claim 1 having an affinity for the sulfonated copolymer of acrylic acid and acrylamide, the monoclonal antibody being bound to a solid carrier.

## Patentansprüche

1. Monoklonaler Antikörper, der eine Affinität für ein sulfoniertes Acrylsäure/Acrylamid-Copolymer aufweist.

2. Monoklonaler Antikörper nach Anspruch 1, wobei der monoklonale Antikörper von einer Hybridom-Zellinie gebildet worden ist.

3. Verfahren zur Herstellung eines monoklonalen Antikörpers nach Anspruch 1, der eine Affinität für ein sulfoniertes Acrylsäure/Acrylamid-Copolymer aufweist, wobei das Verfahren die folgenden Stufen umfaßt:
a) Immunisieren eines Säugetiers mit dem sulfonierten Acrylsäure/Acrylamid-Copolymer;
b) Herstellung einer den monoklonalen Antikörper bildenden Hybridomzelle aus einer Zelle des immunisierten Säugetiers;
c) Klonieren der genannten Hybridomzelle zur Herstellung einer Zellinie; und
d) Extrahieren des genannten monoklonalen Antikörpers aus der genannten Hybridomzellinie.

4. Verfahren nach Anspruch 3, bei dem das Säugetier eine Maus ist.

5. Verfahren zur Bestimmung der Anwesenheit oder der Konzentration eines sulfonierten Acrylsäure/Acrylamid-Copolymers in einer Flüssigkeit, wobei das Verfahren die folgenden Stufen umfaßt:
Inkubieren einer Probe der das sulfonierte Acrylsäure/Acrylamid-Copolymer enthaltenden Flüssigkeit mit einem monoklonalen Antikörper nach Anspruch 1, der eine Affinität für das sulfonierte Acrylsäure/Acrylamid-Copolymer aufweist und an einen festen Träger gebunden ist.

## Revendications

1. Anticorps monoclonal qui présente une affinité pour un copolymère sulfoné d'acide acrylique et d'acrylamide.

2. Anticorps monoclonal selon la revendication 1, dans lequel l'anticorps monoclonal a été formé par une ligne de cellules hybridomes.

3. Procédé pour la réalisation d'un anticorps monoclonal selon la revendication 1, qui présente une affinité pour un copolymère sulfoné d'acide acrylique et d'acrylamide, le procédé comprenant les étapes suivantes:
a) immunisation d'un mammifère avec le copolymère sulfoné d'acide acrylique et d'acrylamide,
b) réalisation d'une cellule hybridome formant l'anticorps monoclonal à partir d'une cellule du mammifère immunisé,
c) clonage de ladite cellule hybridome pour la réalisation d'une ligne cellulaire, et
d) extraction dudit anticorps monoclonal de ladite ligne de cellules hybridomes.

4. Procédé selon la revendication 3, dans lequel le mammifère est une souris.

5. Procédé pour la détermination de la présence ou de la concentration d'un copolymère sulfoné d'acide acrylique et d'acrylamide dans un liquide, le procédé comprenant les étapes suivantes:
incubation d'un échantillon du liquide contenant le copolymère sulfoné d'acide acrylique et d'acrylamide avec un anticorps monoclonal selon la revendication 1 qui présente une affinité pour le copolymère sulfoné d'acide acrylique et d'acrylamide, l'anticorps monoclonal étant lié à un support fixe.
